(19) **Europäisches Patentamt / European Patent Office / Office européen des brevets**

(11) **EP 4 512 327 A1**

(12) # EUROPEAN PATENT APPLICATION

(43) Date of publication:
26.02.2025 Bulletin 2025/09

(21) Application number: 24196008.7

(22) Date of filing: 22.08.2024

(51) International Patent Classification (IPC):
A61B 5/145 (2006.01)   A61B 5/1495 (2006.01)
A61B 5/1486 (2006.01)   A61B 5/00 (2006.01)

(52) Cooperative Patent Classification (CPC):
A61B 5/14532; A61B 5/14865; A61B 5/1495;
A61B 5/6849; A61B 2560/0223; A61B 2560/0247

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 24.08.2023 KR 20230110959

(71) Applicant: I-SENS, INC.
Seoul 06646 (KR)

(72) Inventor: LEE, Da-vid
06646 Seoul (KR)

(74) Representative: HGF
HGF Limited
1 City Walk
Leeds LS11 9DX (GB)

(54) **METHOD FOR CALIBRATING SENSITIVITY FOR GLUCOSE MEASUREMENT**

(57) An embodiment may provide a method for calibrating sensitivity for glucose measurement including: obtaining (S1201) an offset value for determining sensitivity; determining (S1203) a first sensitivity from a sensor data and a reference glucose value, based on the offset value; and determining (S1205) sensitivity by adjusting the first sensitivity, wherein the determining of the sensitivity includes obtaining the first sensitivity, and determining (S1205-1) sensitivity A by adjusting the first sensitivity according to an elapsed period of time after a sensor is inserted into a body; obtaining the first sensitivity, and determining (S1205-1) sensitivity B by adjusting the first sensitivity by reflecting an average sensor data obtained by averaging a plurality of sensor data and an average glucose value obtained by averaging a plurality of glucose values to the first sensitivity; and determining (S1205-2) any one of the sensitivity A and the sensitivity B as the sensitivity.

**FIGURE 12**

**Description**

CROSS-REFERENCE TO RELATED PATENT APPLICATION

The present application claims priority under 35 U.S.C. § 119(a) to Korean patent application number 10-2023-0110959 filed on August 24, 2023, in the Korean Intellectual Property Office, the entire disclosure of which is incorporated by reference herein.

BACKGROUND OF THE INVENTION

1. Field

The present embodiment relate to calibrating sensitivity for glucose measurement, and more specifically, to a technology for calibrating sensitivity including a plurality of steps for adjusting the sensitivity.

2. Description of the Related Art

Recently, with the advancement of medical technology, various medical devices that are attached to a user's body have been developed and sold. A medical device attached to the user's body may be useful for monitoring biometric information or providing treatment by being attached to the skin of a chronic disease patient.

For example, chronic diseases such as diabetes require continuous management, and a medical device that is attached to the skin and measures glucose may be used to monitor glucose in diabetic patients. Diabetes is characterized by almost no noticeable symptoms in the early stages, but as the disease progresses, symptoms characteristic of diabetes appear, such as polydipsia, polyphagia, polyuria, weight loss, general malaise, itchy skin, cases where wounds on the hands and feet do not heal and persist for a long time, and etc. As diabetes progresses further, complications such as vision impairment, high blood pressure, kidney disease, stroke, periodontal disease, muscle spasms, neuralgia, and gangrene appear. In order to diagnose diabetes and manage it to prevent it from developing into complications, systematic glucose measurement and treatment must be carried out together.

For diabetic patients and people who have not developed diabetes but have more sugar than normal detected in their blood, many medical device manufacturers provide various types of glucose meters that may measure glucose.

There are two types of glucose meters, one where the user collects blood from the fingertip and measures glucose on a one-time basis, and one where the user attaches it to the stomach, arm, etc., and continuously measures glucose.

In the case of diabetic patients, they generally go back and forth between hyperglycemia and hypoglycemia, and emergency situations occur during hypoglycemia, and loss of consciousness or prolonged hypoglycemia without sugar supply may result in death. Therefore, immediate detection of hypoglycemia is very important for diabetic patients, but glucose meters that measure glucose intermittently have limitations in accurately detecting this condition.

Recently, to overcome these limitations, a continuous glucose monitoring system (CGMS), which is inserted into the human body and measures glucose levels at intervals of several minutes, has been developed and used. In order to minimize the user's pain and resistance following blood collection, the CGMS may measure glucose continuously after inserting a needle-shaped transdermal sensor into areas where pain is relatively less, such as the stomach, arm, etc.

The CGMS includes a sensor transmitter that is inserted into the user's skin to measure glucose in the body and transmit the measured glucose level, and a terminal that outputs the received glucose level.

Here, since glucose is measured while the sensor transmitter is attached to the body and part of the sensor is inserted into the human body, the sensor inserted into the human body may be recognized as a foreign substance. In other words, a biological reaction to the sensor, for example, a hydration reaction, may occur. As a result, at the beginning when the sensor is inserted, sensitivity (a slope) may change. This may reduce the accuracy of the sensor and make it difficult to maintain the expiration date. The changed slope may reduce the accuracy of the CGMS by calculating excessive or insufficient glucose values for the measured biosignals. Conventionally, in order to compensate for this change in sensitivity (slope), a method using a simple sensitivity divided by a reference glucose value input by the user (for example, a glucose value measured by a blood glucose monitoring system (BGMS)) and a current value of the sensor and a preset offset value, or a method of determining sensitivity through regression analysis of the reference glucose value and a corresponding current value was developed. However, such a method may have a problem of providing inaccurate sensitivity and offset values because it does not respond sensitively to abnormal glucose values or abnormal operation of the sensor.

SUMMARY OF THE INVENTION

Against this background, one object of embodiments of the present disclosure is to provide a technology for calibrating sensitivity in order to calculate or obtain accurate sensitivity.

Against this background, another object of embodiments of the present disclosure is to provide a technology for providing calibrated sensitivity by adjusting the sensitivity through multiple steps to increase the accuracy of the sensitivity.

In order to achieve the above described object, one embodiment may provide a method for calibrating sensitivity for glucose measurement, including, obtaining an offset value for determining sensitivity; determining a first sensitivity from a sensor data and a reference glucose value, based on the offset value; and determining sensitivity by adjusting the first sensitivity, wherein the determining of the sensitivity may include obtaining the first sensitivity, and determining sensitivity A by adjusting the first sensitivity according to an elapsed period of time after a sensor is inserted into a body; obtaining the first sensitivity, and determining sensitivity B by adjusting the first sensitivity by reflecting an average sensor data obtained by averaging a plurality of sensor data and an average glucose value obtained by averaging a plurality of glucose values to the first sensitivity; and determining any one of the sensitivity A and the sensitivity B as the sensitivity.

Another embodiment may provide a method for calibrating sensitivity for glucose measurement, including obtaining an offset value for determining sensitivity; determining a first sensitivity from a sensor data and a reference glucose value, based on the offset value; determining sensitivity A by adjusting the first sensitivity according to an elapsed period of time after a sensor is inserted into a body; determining a second sensitivity from the sensitivity A and a predetermined sensitivity depending on a sensor; determining sensitivity B by adjusting the second sensitivity by reflecting an average sensor data obtained by averaging a plurality of sensor data over a predetermined period of time and an average glucose value obtained by averaging a plurality of glucose values over the predetermined period to the second sensitivity; determining a third sensitivity by adjusting the sensitivity B according to a reliability of the first sensitivity based on a difference between the first sensitivity and a previous sensitivity; determining a fourth sensitivity from the third sensitivity according to whether a glucose value obtained from the offset value and the third sensitivity falls within a specific range; and determining the fourth sensitivity as the sensitivity.

As described above, according to the embodiments, it is possible to accurate sensitivity by calibrating the sensitivity.

In addition, according to the embodiments, it is possible to increase the accuracy of sensitivity by adjusting the sensitivity through multiple steps.

BRIEF DESCRIPTION OF THE DRAWINGS

FIG. 1 is a diagram for schematically explaining a glucose monitoring system according to one embodiment.
FIG. 2 is a diagram for explaining an applicator for attaching a sensor transmitter to a human body according to one embodiment.
FIG. 3 is a diagram for explaining a process of attaching a sensor transmitter to a human body using an applicator according to one embodiment.
FIG. 4 is a configuration diagram of a sensor transmitter according to one embodiment.
FIG. 5 is a configuration diagram of a terminal according to one embodiment.
FIG. 6 is a flowchart illustrating a method for calibrating sensitivity according to one embodiment.
FIG. 7 is an exemplary diagram of adjusting sensitivity based on coding information of a sensor according to one embodiment.
FIG. 8 is an exemplary diagram of adjusting sensitivity based on a difference from a previous sensitivity according to one embodiment.
FIGS. 9 to 11 are exemplary diagrams of adjusting sensitivity based on a specific range formed by a reference glucose value and a previous glucose value obtained from a previous sensitivity according to one embodiment.
FIG. 12 is a flowchart illustrating a first method for calibrating sensitivity according to another embodiment.
FIG. 13 is a flowchart illustrating a method of adjusting sensitivity according to an elapsed period of time after a sensor is inserted into a body according to FIG. 12.
FIG. 14 is an exemplary diagram of adjusting sensitivity according to an elapsed period of time after a sensor is inserted into a body according to FIG. 13.
FIG. 15 is a flowchart illustrating a method of adjusting sensitivity based on an average sensor data and an average glucose value according to FIG. 12.
FIG. 16 is a flowchart illustrating a method of adjusting sensitivity based on an average sensor data and an average glucose value according to FIG. 15.
FIG. 17 is a flowchart illustrating a first example of a first method for calibrating sensitivity according to another embodiment.
FIG. 18 is a flowchart illustrating a second example of a first method for calibrating sensitivity according to another embodiment.
FIG. 19 is a flowchart illustrating a third example of a first method for calibrating sensitivity according to another embodiment.
FIG. 20 is a flowchart illustrating a second method for calibrating sensitivity according to another embodiment.

DETAILED DESCRIPTION

In describing the present disclosure, if it is determined that related known functions may unnecessarily obscure the gist of the present disclosure as they are obvious to those skilled in the art, detailed descriptions thereof will be omitted.

The terms used in the present application are used merely to describe particular embodiments and are not intended to limit the present disclosure. Singular expressions include plural expressions unless the context clearly indicates otherwise. In the present application, terms such as "include" or "have" are intended to designate the presence of features, numbers, steps, operations, components, parts, or combinations thereof described in the specification, and it should be understood that they do not preclude the possibility of the presence or addition of one or more other features, numbers, steps, operations, components, parts, or combinations thereof.

Hereinafter, embodiments according to the present disclosure will be described in detail with reference to the accompanying drawings, and in describing with reference to the accompanying drawings, identical or corresponding components will be assigned the same drawing numbers and overlapping descriptions thereof will be omitted.

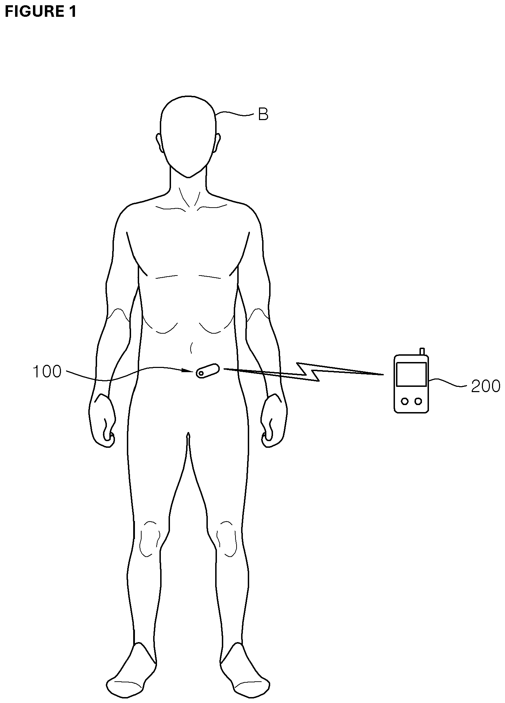

FIG. 1 is a diagram for schematically explaining a glucose monitoring system according to one embodiment.

Referring to FIG. 1, a glucose monitoring system 10 (hereinafter referred to as "system") according to one embodiment may include a sensor transmitter 100 and a terminal 200.

The sensor transmitter 100 is attached to a human body B, and when the sensor transmitter 100 is attached to the human body B, one end of a sensor of the sensor transmitter 100 is inserted into the skin to periodically extract body fluids from the human body and measure glucose.

The terminal 200 may receive a biosignal including glucose information from the sensor transmitter 100, generate glucose information from the biosignal, and output to the user. The terminal 200 may include, but is not limited to, various devices such as smartphones, mobile phones, tablet PCs, desktops, and laptops, and may have a communication interface capable of communicating with the sensor transmitter 100 and may include a device on which a program or application may be installed.

The sensor transmitter 100 may periodically transmit measured biosignals to the terminal 200 at the request of the terminal 200 or at a set time. For data communication between the sensor transmitter 100 and the terminal 200, the sensor transmitter 100 and the terminal 200 may be connected to each other via a wired connection such as a USB cable or wirelessly using methods such as infrared communication, NFC communication, or Bluetooth.

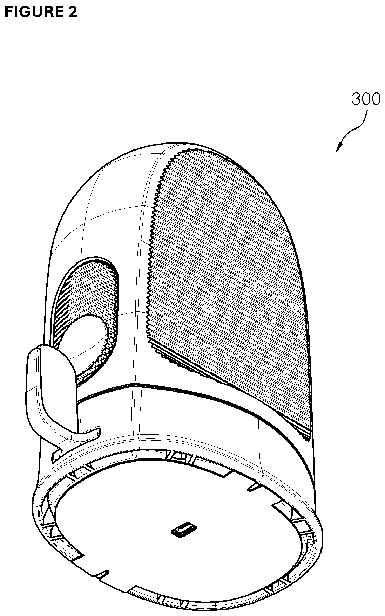

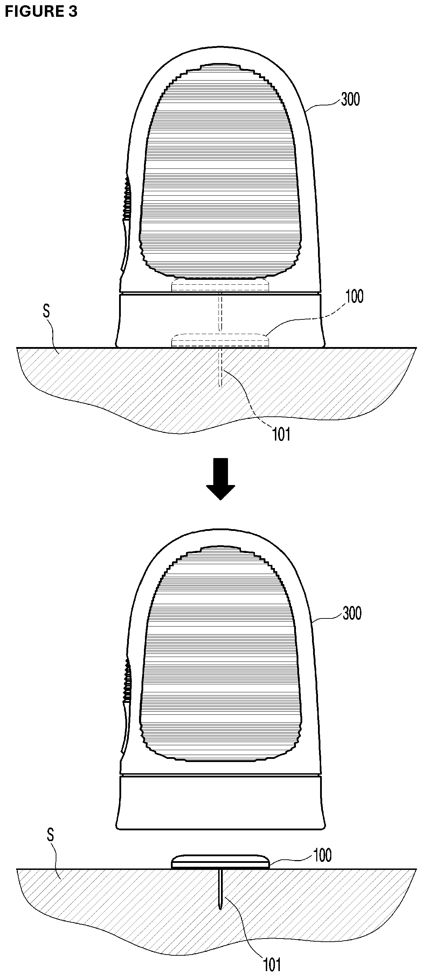

FIG. 2 is a diagram for explaining an applicator for attaching a sensor transmitter to a human body according to one embodiment, and FIG. 3 is a diagram for explaining a process of attaching a sensor transmitter to a human body using an applicator according to one embodiment.

Referring to FIGS. 2 and 3, an applicator 300 according to one embodiment has a sensor transmitter 100 inside and operates to discharge the sensor transmitter 100 to the outside and attach it to a specific body part of the user through manipulation by the user. The applicator 300 is formed in a shape with one side open, and the sensor transmitter 100 is installed in the applicator 300 through the open side of the applicator 300.

When attaching the sensor transmitter 100 to a part of the body using the applicator 300, in order to insert one end of a sensor provided in the sensor transmitter 100 into the skin, the applicator 300 may include a needle (not shown) formed to surround one end of the sensor inside, a first elastic member (not shown) that pushes the needle and one end of the sensor together into the skin, and a second elastic member (not shown) for pulling out only the needle. Through this configuration of the applicator 300, the needle and one end of the sensor may be simultaneously inserted into the skin by decompressing the first elastic member (not shown) disposed in a compressed state inside the applicator 300. When one end of the sensor is inserted into the skin, only the needle is pulled out by decompressing the compressed second elastic member (not shown). The user may safely and easily attach the sensor transmitter 100 to the skin through the applicator 300.

Looking in detail at the process of attaching the applicator 300 to the human body B, with a protective cap (not shown) removed, the open side of the applicator 300 is brought into close contact with the skin S of a specific area of the human body B. When the applicator 300 is operated in this way while the applicator 300 is in close contact with the skin S of the human body B, the sensor transmitter 100 is discharged from the applicator 300 and may be attached to the skin S. Here, one end of the sensor 101 is disposed at the lower part of the sensor transmitter 100, exposed from the sensor transmitter 100, and one end of the sensor 101 may be partially inserted into the skin S through the needle provided in the applicator 300. Therefore, the sensor transmitter 100 may be attached to the skin S with one end of the sensor 101 inserted into the skin S.

Here, an adhesive tape may be provided on the surface of the sensor transmitter 100 in contact with the human body B so that the sensor transmitter 100 may be fixedly attached to the skin S of the human body B. Therefore, when the applicator 300 is separated from the skin S of the human body B, the sensor transmitter 100 may be fixedly attached to the skin S of the human body B by the adhesive tape.

Afterwards, when power is applied to the sensor transmitter 100, the sensor transmitter 100 communicates with the terminal, and the sensor transmitter 100 may transmit biosignals including glucose information to the terminal. The sensor transmitter 100 may generate not only glucose information but also various biometric information, and hereinafter, it will be explained that glucose information is measured as an example of biometric information.

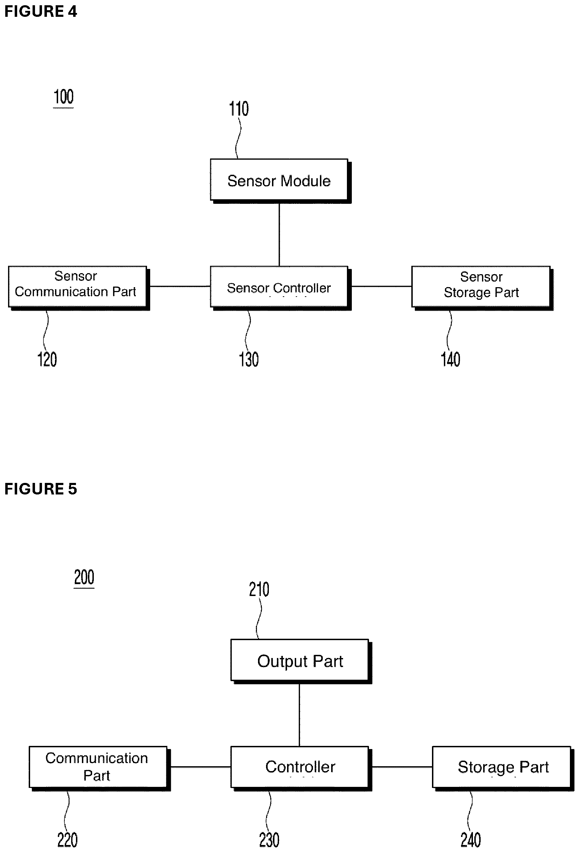

FIG. 4 is a configuration diagram of a sensor transmitter according to one embodiment.

Referring to FIG. 4, a sensor transmitter 100 according to one embodiment may include a sensor module 110, a sensor communication part 120, a sensor controller 130, and a sensor storage part 140.

The sensor module 110 may include at least one sensor that is inserted into the human body and senses biomass. At least one sensor may measure biomass and generate biosignals. The biosignal is an analog signal and may include a current value.

The sensor communication part 120 may exchange data or information with the terminal. For example, the sensor communication part 120 may transmit biosignals received from the sensor module 110 or data stored in the sensor storage part 140 to the terminal.

The sensor controller 130 may control the overall configuration of the sensor transmitter 100, including the sensor module 110, the sensor storage part 140, and the sensor communication part 120. For example, the sensor controller 130 may receive a control signal from the terminal and control the configuration of the sensor transmitter 100 accordingly. In addition, the sensor controller 130 may process biosignals. For example, the sensor controller 130 may convert biosignals into analog or digital form or perform processing to remove noise as needed.

Data or information may be stored in the sensor storage part 140. For example, the sensor storage part 140 may store data on biomass measured by the sensor module 110, for example, the current value of the biosignal, or data received from the terminal, for example, the command value of the control signal.

FIG. 5 is a configuration diagram of a terminal according to one embodiment.

Referring to FIG. 5, the terminal 200 according to one embodiment may include an output part 210, a communication part 220, a controller 230, and a storage part 240.

The output part 210 may output biometric information included in the biosignal, for example, glucose information, so that the user may check it. For example, the output part 210 may display glucose information as a numerical level (value) or a graph processed from the numerical value.

The communication part 220 may communicate with the sensor communication part of the sensor transmitter and exchange data or information. For example, the communication part 220 may receive a biosignal containing information about biomass measured by the sensor transmitter (i.e., biometric information). Here, the communication part 220 may receive primarily processed biosignals from the sensor transmitter. Alternatively, the communication part 220 may transmit a control signal for controlling the sensor transmitter to the sensor transmitter.

Data or information may be stored in the storage part 240. For example, data received from the sensor transmitter, for example, biometric information, may be stored in the storage part 240. Here, the biometric information includes glucose information and may have the form of a current value. Alternatively, data input from the user or environment setting data for setting the operating environment of the terminal may be stored in the storage part 240.

The controller 230 may include at least one processor that executes a program for calibrating sensitivity for glucose measurement and at least one memory in which the program is stored. The memory and processor included in the controller 230 may be integrated into one chip or may be physically separated.

Memory may be implemented as non-volatile memory devices such as read only memory (ROM), programmable ROM (PROM), erasable programmable ROM (EPROM), electrically erasable programmable ROM (EEPROM), and flash memory, or volatile memory devices such as random access memory (RAM) to store various programs, data, and/or information.

Meanwhile, the controller 230 may generate a glucose value, which is quantified glucose information, from biometric information. To this end, the controller 230 may obtain biometric information in the form of a current value from the sensor transmitter, and preprocess and/or process the current value of the biometric information. The controller 230 may first obtain the sensitivity and generate the glucose value based on this sensitivity.

The controller 230 may determine the sensitivity based on the current value and the reference glucose value input from the outside, and the sensitivity may be determined by Equation 1 below. Here, C refers to the current value at the point where sensitivity is determined based on the reference glucose value, i.e., when sensitivity is calibrated, and BGr is the reference glucose value obtained from the BGMS and does not necessarily need to be obtained at the point of sensitivity calibration.

[Equation 1]

$$S = \frac{BGr}{C}$$

After the sensitivity is obtained as above, the controller 230 may multiply the current value and this sensitivity to generate a glucose value corresponding to the current value. The controller 230 may provide this new glucose value to the user through the output part 240. For example, the controller 230 may obtain the sensitivity as shown in Equation 1 through a first current value obtained at a first point and one reference glucose value, and obtain a new glucose value using this sensitivity until the point of next sensitivity calibration (i.e., a second point). In addition, the controller 230 may calibrate the sensitivity again at the second point. The controller 230 may obtain the sensitivity as shown in Equation 1 through a second current value obtained at the second point and another reference glucose value and newly obtain a glucose value corresponding thereto.

Here, this sensitivity may fluctuate due to various environmental variables. For example, fluctuations in current values or a negative intercept phenomenon in which the current value continues to decrease that occur due to the hydration effect that occurs when the sensor is inserted into the body may be factors that change sensitivity. When the sensor transmitter sends a current value lower than the original to the terminal 200 due to the negative intercept phenomenon, the controller 230 of the terminal 200 may obtain the sensitivity from the current value smaller than the original according to Equation 1. The sensitivity obtained in this way may be very large. If the controller 230 obtains the glucose value with excessively high sensitivity, the glucose value may increase even with a small current value and the controller 230 may generate an inaccurate glucose value.

To correct this error, the controller 230 may obtain an offset value and reflect this offset value in obtaining sensitivity. The controller 230 may obtain the offset value in various ways without limitation, and the offset value may be fixed or variable. The controller 230 may determine sensitivity reflecting the offset value according to Equation 2 below. Here, Voff is the offset value and may be a value predetermined by the user or a value that may be changed by the algorithm of the storage part 250.

[Equation 2]

$$S = \frac{BGr}{C - Voff}$$

In the above described example, the decrease in current value due to the negative intercept phenomenon is offset by the offset value, and the smaller denominator becomes larger, so that the sensitivity may also be changed to an appropriate level that is not excessive. However, the controller 230 of the terminal 200 according to one embodiment does not immediately use the sensitivity reflecting the offset value to obtain the glucose value, but may adjust the sensitivity and ultimately use the adjusted sensitivity to obtain the glucose value. Hereinafter, a method in which the controller 230 of the terminal 200 adjusts the sensitivity through a number of steps to adjust the sensitivity will be described.

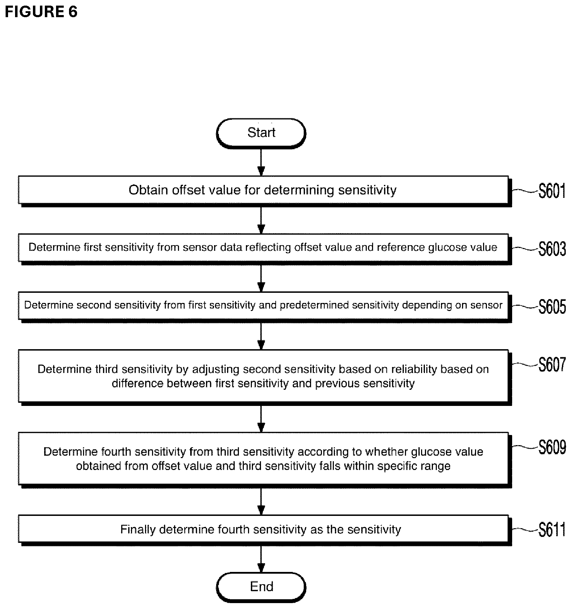

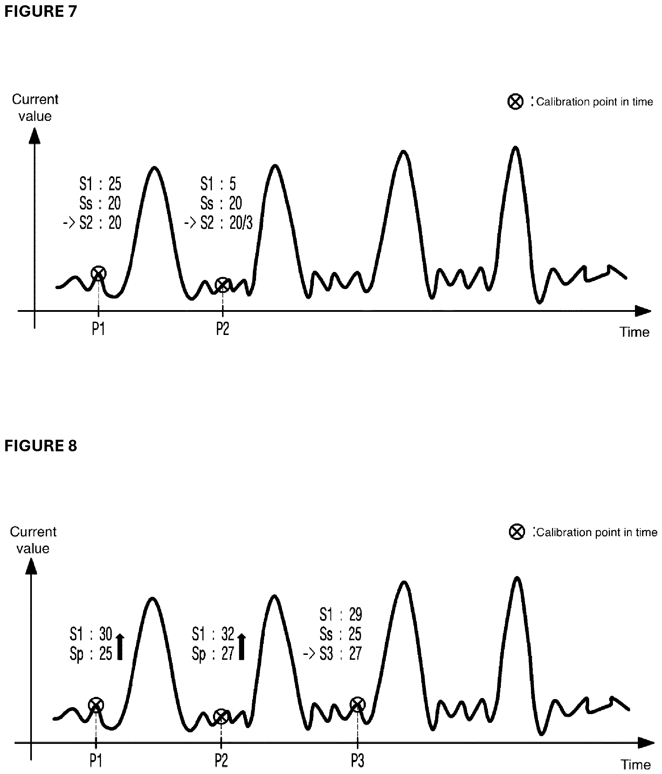

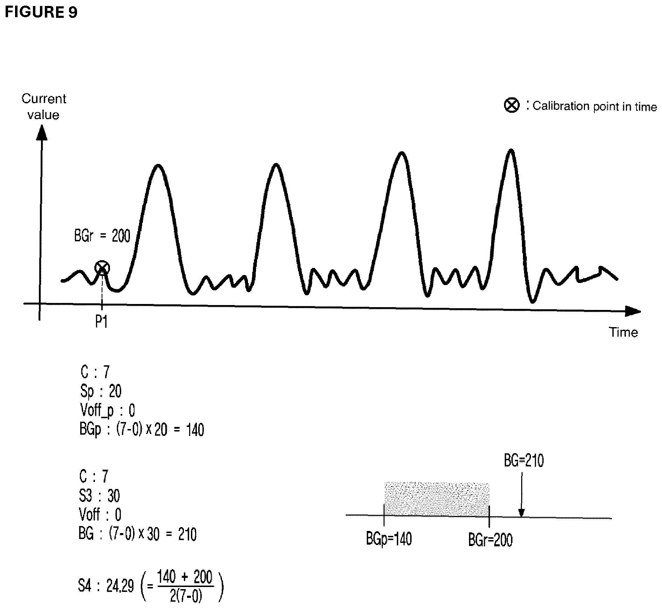

FIG. 6 is a flowchart illustrating a method for calibrating sensitivity according to one embodiment, FIG. 7 is an exemplary diagram of adjusting sensitivity based on coding information of a sensor according to one embodiment, FIG. 8 is an exemplary diagram of adjusting sensitivity based on a difference from a previous sensitivity according to one embodiment, and FIGS. 9 to 11 are exemplary diagrams of adjusting sensitivity based on a specific range formed by a reference glucose value and a previous glucose value obtain from a previous sensitivity according to one embodiment.

Referring to FIG. 6, a method for calibrating sensitivity in a terminal that constitutes a system according to one embodiment may be shown. Hereinafter, sensitivity calibration is described as being performed in the terminal, but is not limited thereto, and depending on the configuration, may also be performed on the sensor transmitter or a host connected to the terminal through a wired or wireless network, for example, a server. The controller of the terminal may first determine the sensitivity required to generate a glucose value from the current value and generate the glucose value by applying the current value to the finally determined sensitivity.

To this end, the controller of the terminal may first obtain an offset value for determining sensitivity (step S601). The offset value may be a value that compensates for changes in sensitivity due to a decrease in current value.

In addition, the controller may determine the sensitivity from the current value reflecting the offset value and the reference glucose value according to Equation 2 (step S603). The sensitivity determined here is not the final sensitivity, but may simply be a sensitivity that reflects only the offset value before going through an adjustment step. Hereinafter, for convenience of explanation, this sensitivity will be referred to as 'first sensitivity'. In addition, the current value is a type of value measured by the sensor included in the sensor module and may also be called a sensor data.

First, an adjustment to the first sensitivity may be performed. The controller of the terminal may determine a second sensitivity from the first sensitivity and a predetermined sensitivity depending on the sensor (step S605). The predetermined sensitivity depending on the sensor may be a preset sensitivity for any one sensor, or may be a preset sensitivity for any one group (or a lot) which is a collection of a plurality of sensors. The sensitivity of one group of sensors may be understood as a specific sensitivity representing the entire one group, not just one sensor. Hereinafter, for convenience of explanation, the predetermined sensitivity depending on the sensor may be referred to as 'sensor sensitivity'.

Here, sensor sensitivity may include ideal sensitivity expected from one sensor or one group of sensors under ideal conditions. For example, sensor sensitivity may be understood as the sensitivity that one sensor or one group of sensors should have under ideal conditions, such as a laboratory environment. Alternatively, sensor sensitivity may be understood as a sensitivity predetermined at the factory when one sensor or one group of sensors is manufactured. When the terminal is used, it needs to be coded for initialization, and the sensor sensitivity may be included in the coding information during the coding process and input and/or stored in the terminal.

Referring to FIG. 7, an example of determining the second sensitivity from the first sensitivity and sensor sensitivity may be shown. The controller of the terminal may reflect the sensor sensitivity to adjust the first sensitivity and determine the second sensitivity as a result. However, the controller of the terminal may reflect sensor sensitivity differently in some cases.

First, the controller may compare the first sensitivity and the sensor sensitivity. If the first sensitivity is greater than the sensor sensitivity, the controller may determine the sensor sensitivity as the second sensitivity. For example, in the present drawing, if the first sensitivity (S1) at a first calibration point in time (P1) is 25 and the sensor sensitivity (Ss), which is the sensitivity based on coding information, is 20, the controller may determine the second sensitivity (S2) to be 20. Hereinafter, for convenience of explanation, units are omitted. The controller may trust the sensor sensitivity (Ss) more and determine the sensor sensitivity (Ss) as the second sensitivity (S2).

Otherwise, i.e., if the first sensitivity is less than the sensor sensitivity, the controller may process the sensor sensitivity and determine the processed second sensitivity as the first sensitivity. For example, in the present drawing, if the first sensitivity (S1) at a second calibration point in time (P2) is 5 and the sensor sensitivity (Ss), which is the sensitivity based on the coding information of the sensor, is 20, the controller may process the sensor sensitivity (Ss) and obtain a value corresponding to 1/3 of the sensor sensitivity (Ss), and determine 20/3 (≒6.7), which is a value corresponding to 1/3 of the sensor sensitivity (Ss), as the second sensitivity (S2). The controller may trust the sensor sensitivity (Ss) more and determine the processed value of the sensor sensitivity (Ss) as the second sensitivity (S2).

If the first sensitivity is the same as the sensor sensitivity, the controller may determine the first sensitivity as the second sensitivity.

Returning again to FIG. 6, an adjustment to the second sensitivity may be performed. The controller of the terminal may determine a third sensitivity by adjusting the second sensitivity based on the reliability of the first sensitivity based on the difference between the first sensitivity and a previous sensitivity (step S607). Here, the previous sensitivity refers to the final sensitivity at a previous point, and the previous point may refer to a specific point in the past before the current point in time at which the sensitivity is to be calibrated. The controller of the terminal may have obtained the glucose value according to the previous sensitivity at the previous point. Hereinafter, for convenience of explanation, it may be referred to as 'previous sensitivity.'

Specifically, if the first sensitivity is continuously higher or lower than the previous sensitivity (at least twice or more), the controller of the terminal may determine that the first sensitivity is trustworthy and that the reliability of the first sensitivity is high. Then, the controller may obtain the combined sensitivity reflecting the first sensitivity and the second sensitivity and determine this combined sensitivity as the third sensitivity. The controller may obtain the combined sensitivity by averaging the first sensitivity and the second sensitivity.

Referring to FIG. 8, an example of determining the third sensitivity by adjusting the second sensitivity may be shown. For example in the present drawing, if the first sensitivity (S1) is 30 and the previous sensitivity (Sp) is 25 at the first calibration point (P1), and the first sensitivity (S1) is 32 and the previous sensitivity (Sp) is 27 at the second calibration point (P2), since the first sensitivity (S1) has a higher value than the previous sensitivity (Sp) at two consecutive calibration points, the controller may determine that the reliability of the first sensitivity (S1), which is 29 at a third calibration point in time (P3), is high. Then, at the third calibration point (P3), the controller may obtain the combined sensitivity from the first sensitivity (S1) 29 and the second sensitivity (S2) 25. The controller may obtain 27 (=(29+25)/2), which is the average value of the first sensitivity (S1) 29 and the second sensitivity (S2) 25, as the combined sensitivity. The controller may determine the combined sensitivity 27 as the third sensitivity (S3).

As such, if the first sensitivity is continuously high or low, the first sensitivity may be considered reasonable, and unlike step S605, it is preferable that the first sensitivity is reflected more than the second sensitivity.

Returning again to FIG. 6, an adjustment to the third sensitivity may be performed. The controller of the terminal may obtain the glucose value from the offset value and the third sensitivity, and determine a fourth sensitivity from the third sensitivity according to whether the obtained glucose value falls within a specific range. If the obtained glucose value falls within the specific range, the controller may consider the third sensitivity to be appropriate and determine the third sensitivity as the fourth sensitivity. Otherwise, the controller may consider the third sensitivity to be inappropriate, obtain an adjustment sensitivity instead of the third sensitivity, and determine the adjustment sensitivity as the fourth sensitivity.

Here, the specific range may be specified by the previous glucose value and the reference glucose value. The controller may obtain the previous glucose value from a current value at the current point in time, previous sensitivity, and previously obtained offset value (i.e. previous offset value). In addition, the controller may obtain the reference glucose value (i.e. the reference glucose value used to determine the first sensitivity in step S601) from the outside. The controller may determine whether the glucose value obtained from the current value at the current point in time, the offset value and the third sensitivity at the current point in time is in the area defined by the previous glucose value and the reference glucose value.

Referring to FIG. 9, one example of determining the fourth sensitivity from the third sensitivity according to whether the glucose value obtained from the third sensitivity falls within the specific range may be shown. In one example, it may be assumed that the controller determines the fourth sensitivity at the first calibration point (P1). First, the controller may specify the specific range and determine the effectiveness of the third sensitivity.

If the current value (C) at the first calibration point (P1) is 7, the previous sensitivity (Sp) is 20, and the previous offset value (Voff_p) is 0, the previous glucose value (BGp) may be 140 (=(7-0)×20). Here, the previous sensitivity (Sp) and the previous offset value (Voff_p) may be the sensitivity and offset value determined before sensitivity calibration is performed at the first calibration point (P1). If the reference glucose value (BGr) at the first calibration point (P1) is 200, the area of glucose value for which the glucose value according to the third sensitivity is valid, i.e., a specific range specified by the previous glucose value and the reference glucose value, may correspond to 140 to 200.

In addition, since the current value (C) of the first calibration point (P1) is 7, the offset value (Voff) of the first calibration point (P1) is 0, and the third sensitivity (S3) is 30, the controller may obtain glucose value (BG) 210 (= (7-0)×30) therefrom. Since the glucose value (BG) is 210, which is outside the specific range of 140 to 200, the controller may consider the third sensitivity (S3) as invalid and obtain the adjustment sensitivity to determine the fourth sensitivity.

The controller may obtain the adjustment sensitivity using the current value (at the current point in time), the offset value (at the current point in time), the previous glucose value, and the reference glucose value through Equation 3 below.

[Equation 3]

$$Sa = \frac{BGp + BGr}{2} \frac{1}{C - Voff}$$

According to the present example, the current value (C) of the first calibration point (P1) is 7, the offset value (Voff) of the first calibration point (P1) is 0, the previous glucose value (BGp) is 140, and the reference glucose value (BGr) is 200, so the adjustment sensitivity (Sa) may be 24.29. The controller may determine the adjustment sensitivity (Sa) as the fourth sensitivity. The controller may determine 24.29 as the fourth sensitivity.

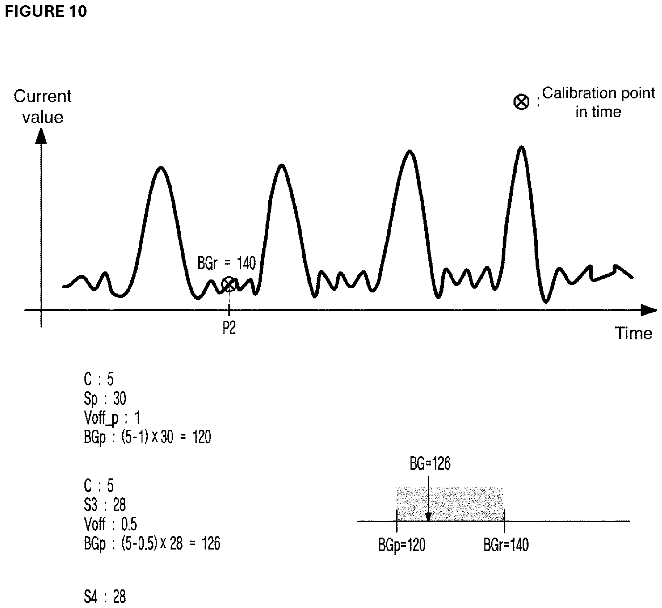

Referring to FIG. 10, another example of determining the fourth sensitivity from the third sensitivity according to whether the glucose value obtained from the third sensitivity falls within a specific range may be shown. In another example, it may be assumed that the controller determines the fourth sensitivity at the second calibration point (P2). First, the controller may specify the specific range and determine the effectiveness of the third sensitivity.

If the current value (C) at the second calibration point (P2) is 5, the previous sensitivity (Sp) is 30, and the previous offset value (Voff_p) is 1, the previous glucose value (BGp) may be 120 (=(5-1)×30). Here, the previous sensitivity (Sp) and previous offset value (Voff_p) may be sensitivity and offset values determined before sensitivity calibration is performed at the second calibration point (P2). If the reference glucose value (BGr) at the second calibration point (P2) is 140, the area of glucose value for which the glucose value according to the third sensitivity is valid, i.e., a specific range specified by the previous glucose value and the reference glucose value, may correspond to 120 to 140.

In addition, since the current value (C) of the second calibration point (P2) is 5, the offset value (Voff) of the second calibration point (P2) is 0.5, and the third sensitivity (S3) is 28, the controller may obtain glucose value (BG) 126 (=(5-0.5)×28) therefrom. Since the glucose value (BG) is 126, which falls within the specific range of 120 to 140, the controller may view the third sensitivity (S3) as valid and determine as the fourth sensitivity. Therefore, in the present example, the fourth sensitivity may be 28.

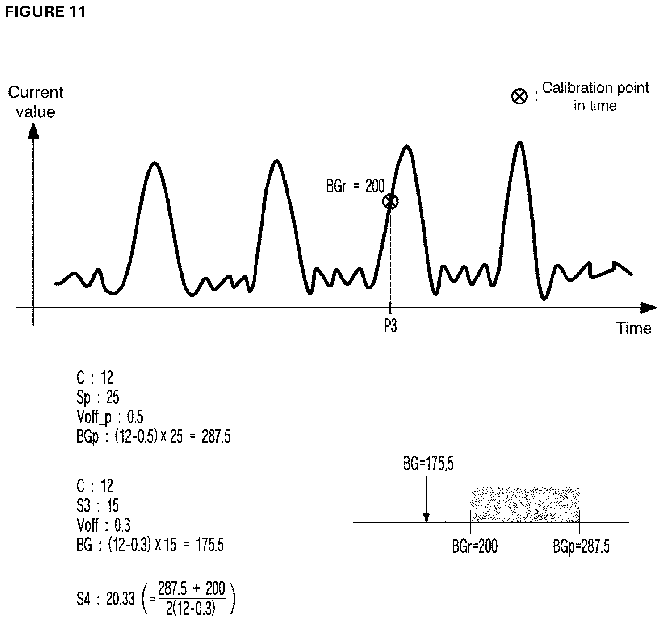

Referring to FIG. 11, another example of determining the fourth sensitivity from the third sensitivity according to whether the glucose value obtained from the third sensitivity falls within a specific range may be shown. In another example, it may be assumed that the controller determines the fourth sensitivity at the third calibration point (P3). First, the controller may specify the specific range and determine the effectiveness of the third sensitivity.

If the current value (C) at the third calibration point (P3) is 12, the previous sensitivity (Sp) is 25, and the previous offset value (Voff_p) is 0.5, the previous glucose value (BGp) may be 287.5 (=(12-0.5)×25). Here, the previous sensitivity (Sp) and previous offset value (Voff_p) may be sensitivity and offset values determined before sensitivity calibration is performed at the third calibration point (P3). If the reference glucose value

(BGr) at the third calibration point (P3) is 200, the area of glucose value for which the glucose value according to the third sensitivity is valid, i.e., a specific range specified by the previous glucose value and the reference glucose value, may correspond to 200 to 287.5.

In addition, since the current value (C) of the third calibration point (P3) is 12, the offset value (Voff) of the third calibration point (P3) is 0.3, and the third sensitivity (S3) is 15, the controller may obtain glucose value (BG) 175.5(=(12-0.3)×15) therefrom. Since the glucose value (BG) is 175.5, which is outside the specific range of 200 to 287.5, the controller may consider the third sensitivity (S3) as invalid and calculate the adjustment sensitivity to determine the fourth sensitivity.

The controller may obtain the adjustment sensitivity through Equation 3 described above, using the current value (at the current point in time), the offset value (at the current point in time), the previous glucose value, and the reference glucose value. According to the present example, the current value (C) of the third calibration point (P3) is 12, the offset value (Voff) of the third calibration point (P3) is 0.3, the previous glucose value (BGp) is 287.5, and the reference glucose value (BGr) is 200, so the adjustment sensitivity (Sa) may be 20.33. The controller may determine 20.33 as the fourth sensitivity.

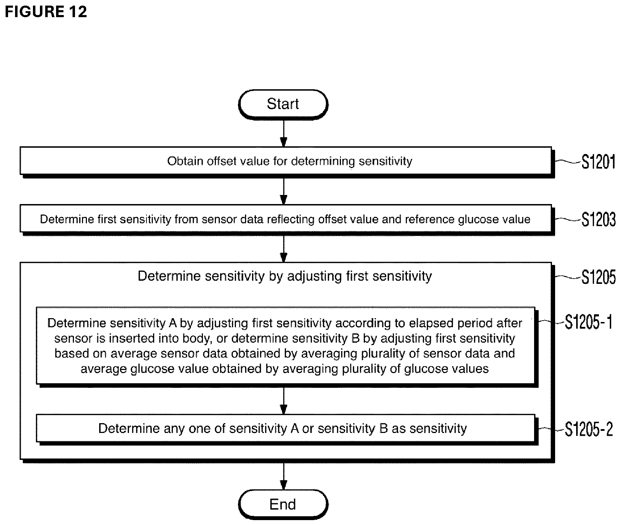

FIG. 12 is a flowchart illustrating a first method for calibrating sensitivity according to another embodiment.

Referring to FIG. 12, a first method of calibrating sensitivity in a terminal that constitutes a system according to another embodiment may be shown. Hereinafter, without limitation, depending on the configuration, it may also be performed on the sensor transmitter or a host connected to the terminal through a wired or wireless network (for example, a server). The controller of the terminal may first determine the sensitivity required to generate a glucose value from the current value and generate the glucose value by applying the current value to the finally determined sensitivity. In addition, other embodiments include the same configuration as the one embodiment, so the description of one embodiment is applied as is, and hereinafter, descriptions that are common to one embodiment will be omitted and the description will focus on differences.

First, the controller of the terminal may first obtain an offset value for determining sensitivity (step S1201). The offset value may be a value that compensates for changes in sensitivity due to a decrease in current value.

In addition, the controller may determine the sensitivity from the current value reflecting the offset value and the reference glucose value according to Equation 2 (step S1203). The sensitivity determined here is a 'first sensitivity' and not the final sensitivity, and may simply be a sensitivity that reflects only the offset value before going through the adjustment step.

Then, an adjustment to the first sensitivity may be performed. The controller of the terminal may adjust the first sensitivity to finally determine the sensitivity (step S1205). In order to finally determine the sensitivity from the first sensitivity, the controller of the terminal may perform the following detailed adjustment.

Specifically, the controller of the terminal may adjust the first sensitivity according to the elapsed period of time after the sensor is inserted into the body, or adjust the first sensitivity based on the average sensor data obtained by averaging the plurality of sensor data and the average glucose value obtained by averaging the plurality of glucose values (step S1205-1). Here, if the first sensitivity is adjusted according to the elapsed period of time after the sensor is inserted into the body, the adjusted first sensitivity may be named 'sensitivity A.' In addition, if the first sensitivity is adjusted based on the average sensor data obtained by averaging the plurality of sensor data and the average glucose value obtained by averaging the plurality of glucose values, the adjusted first sensitivity may be named 'sensitivity B.'

The controller of the terminal may determine any one of sensitivity A and sensitivity B as the adjusted first sensitivity, i.e., the final sensitivity desired by the controller of the terminal (step S1205-2). Here, the controller of the terminal may adjust the first sensitivity by selectively applying any one of the two methods described above. Alternatively, the controller of the terminal may adjust the first sensitivity by sequentially applying both methods described above. Through this step, any one of sensitivity A and sensitivity B may be determined from the first sensitivity, and any one of sensitivity A and sensitivity B may be determined as the final sensitivity for obtaining the glucose value from the current value.

For example, in the case of selective application, the controller of the terminal may generate sensitivity A from the first sensitivity and determine the generated sensitivity A as the final sensitivity. Alternatively, the controller of the terminal may generate sensitivity B from the first sensitivity and determine the generated sensitivity B as the final sensitivity. As another example, in the case of sequential application, the controller of the terminal may generate sensitivity A from the first sensitivity, generate sensitivity B from sensitivity A, and determine the generated sensitivity B as the final sensitivity. Alternatively, sensitivity B may be generated from the first sensitivity of the terminal, sensitivity A may be generated from sensitivity B, and the generated sensitivity A may be determined as the final sensitivity. Hereinafter, the steps for determining sensitivity A and the steps for determining sensitivity B will be discussed in detail.

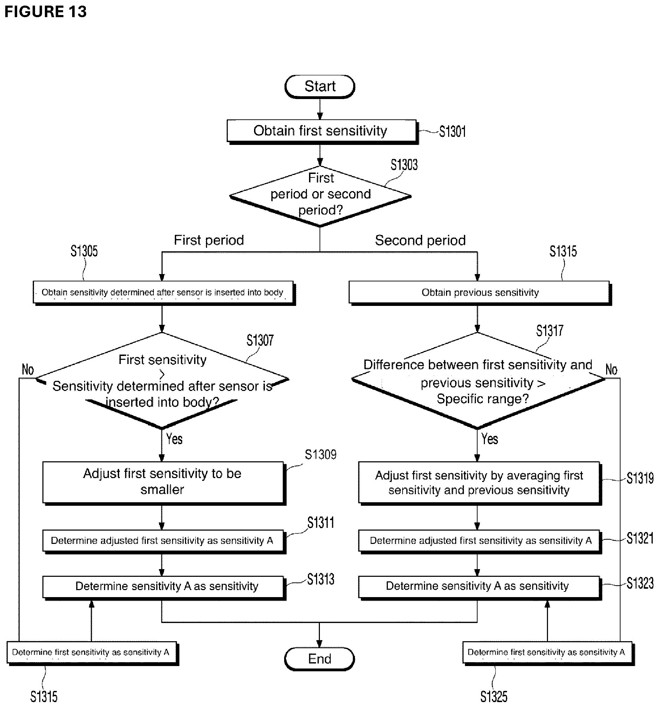

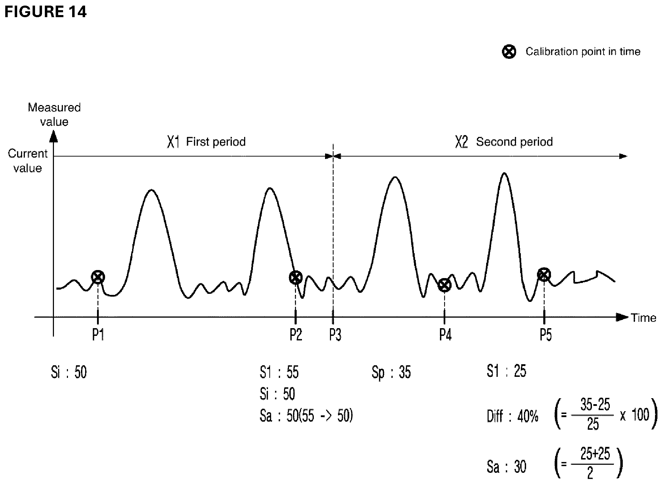

FIG. 13 is a flowchart illustrating a method of adjusting sensitivity according to an elapsed period of time after a sensor is inserted into a body according to FIG. 12, and FIG. 14 is an exemplary diagram of adjusting sensitivity according to an elapsed period of time after a sensor is inserted into a body according to FIG. 13.

Referring to FIG. 13, a method of adjusting sensitivity according to an elapsed period of time after a sensor is inserted into a body according to another embodiment, i.e., a method of determining sensitivity A, may be shown.

The controller of the terminal may generate the first sensitivity from Equation 2 (step S1301). In addition, the controller of the terminal may determine how much time has elapsed after the sensor is inserted into the body. It may be determined whether the current point in time at which sensitivity is to be determined belongs to a first period or a second period (step S1303). The first period and the second period may be distinguished based on whether the hydration process of the sensor occurs. Thus, the first period may refer to the period of time from when the sensor is inserted into the body to the end of the hydration process (i.e. the period of time during which the hydration process exists) and the second period may refer to a period after the end of the hydration process (i.e., a period of time in which the hydration process does not exist). The first and second periods may be distinguished based on the end of the hydration process. The point in time of distinguishing the first and second periods (i.e., the point in time of the end of the hydration process) may be determined based on the change pattern of the current value or may be set in advance by the user for a certain amount of time. The first sensitivity may be adjusted in different ways depending on whether the current point in time belongs to the first period or the second period.

First, if the controller of the terminal determines that the current point in time is within the first period, the first sensitivity may be adjusted based on the sensitivity determined after the sensor is inserted into the body. To this end, the controller of the terminal may obtain the sensitivity determined after the sensor is inserted into the body (step S1305). Preferably, the sensitivity determined after the sensor is inserted into the body may be the sensitivity determined for the first time after the sensor is inserted. In the early days (within 2 to 3 days) after the sensor is inserted, the current value may increase due to the hydration process, and sensitivity may tend to decrease. However, if the first sensitivity is greater than the sensitivity determined after the sensor is inserted, the first sensitivity is considered abnormal and the first sensitivity needs to be adjusted lower to match the sensitivity determined after the sensor is inserted. Therefore, the sensitivity determined 'initially' after insertion of the sensor is derived during the hydration process, making the need for adjustment of the first sensitivity more valid.

In addition, the controller of the terminal may determine whether the first sensitivity exceeds the sensitivity determined after insertion into the body (step S1307). If the first sensitivity exceeds the sensitivity determined after insertion into the body, the controller of the terminal may adjust the first sensitivity to be smaller (YES in step S1307 and step S1309). Preferably the first sensitivity may be adjusted to be as small as the 'initially' determined sensitivity after insertion into the body. Then, the controller of the terminal may determine the adjusted first sensitivity as sensitivity A (step S1311). Sensitivity A is determined as the final sensitivity, and the controller of the terminal may obtain the glucose value for the current value through sensitivity A (step S1313).

If the first sensitivity does not exceed the sensitivity determined after insertion into the body, the controller of the terminal may determine the first sensitivity as sensitivity A without adjusting the first sensitivity (NO in step S1307 and step S1315). Sensitivity A is determined as the final sensitivity, and the controller of the terminal may obtain the glucose value for the current value through sensitivity A (step S1313).

On the other hand, if the controller of the terminal determines that the current point in time is within the second period, the first sensitivity may be adjusted based on the sensitivity determined before the current point in time. To this end, the controller of the terminal may obtain the sensitivity previously determined (i.e., the previous sensitivity) (before the current point in time) (step S1315). Since the second period is a period of time in which the hydration process has ended and no longer occurs, the same adjustment as in the first period may not be required. In the second period, a 'stabilization' section appears where there is almost no change in the current value, so it may be reasonable to say that there is almost no difference between the previous sensitivity and the first sensitivity at the current point in time. Therefore, if the difference in the first sensitivity is large compared to the previous sensitivity (at the current point in time), it may be appropriate to adjust it to get closer to the previous sensitivity.

In addition, the controller of the terminal may determine whether the first sensitivity exceeds the previous sensitivity. To this end, the controller of the terminal may determine whether the difference between the first sensitivity and the previous sensitivity exceeds a specific range (step S1317). If the difference between the first sensitivity and the previous sensitivity exceeds the specific range, the controller of the terminal may adjust the first sensitivity so that the difference falls within the specific range. To this end, the controller of the terminal may adjust the first sensitivity by averaging the first sensitivity and the previous sensitivity (YES in step S1317 and step S1319). In addition, the controller of the terminal may determine the average value as the adjusted first sensitivity. Then, the controller of the terminal may determine the adjusted first sensitivity as sensitivity A (step S1321). Sensitivity A is determined as the final sensitivity, and the controller of the terminal may obtain the glucose value for the current value through sensitivity A (step S1323).

If the difference between the first sensitivity and the previous sensitivity does not exceed the specific range, the controller of the terminal may determine the first sensitivity as sensitivity A without adjusting the first sensitivity (NO in step S1317 and step S1325). Sensitivity A is determined as the final sensitivity, and the controller of the terminal may obtain the glucose value for the current value through sensitivity A (step S1323).

Referring to FIG. 14, an example of adjusting sensitivity according to an elapsed period of time after a sensor is inserted into a body according to another embodiment may be shown. The controller of the terminal may adjust the first sensitivity in different ways depending on a period of time during which the sensor is inserted.

First, looking at the way the first sensitivity is adjusted during the first period (X1) in which the hydration process occurs after the sensor is inserted, the first calibration point (P1) may be the point at which the sensitivity is determined 'for the first time' after the sensor is inserted. The sensitivity determined at the first calibration point (P1), i.e., the sensitivity (Si) initially determined after the sensor is inserted, may be 50. For convenience of explanation, units are omitted. Thereafter, the second calibration point (P2) may be the point at which the controller of the terminal attempts to determine the final sensitivity. At the second calibration point (P2), the controller of the terminal may obtain a first sensitivity (S1) of 55 and determine that the first sensitivity (S1) exceeds the sensitivity (Si) initially determined after the sensor is inserted. The first sensitivity (S1) is adjusted from 55 to 50, and this adjusted first sensitivity may be sensitivity A (Sa). Therefore, the sensitivity A (Sa) in the first period (X1) may be 50.

In addition, the hydration process may end at the third calibration point (P3). After the third calibration point (P3), the second period (X2) may begin.

Next, looking at the way the first sensitivity is adjusted during the second period (X2) when the hydration process ends and the current value stabilizes, a fourth calibration point in time (P4) may be the point corresponding to the previously determined sensitivity (i.e., the previous sensitivity). The controller of the terminal, which aims to determine the sensitivity at a fifth calibration point in time (P5), may use the sensitivity determined at the fourth calibration point (P4). The sensitivity determined at the fourth calibration point (P4), i.e., the previous sensitivity (Sp), may be 35. Subsequently, at the fifth calibration point (P5), the controller of the terminal may obtain a first sensitivity (S1) of 25 and determine that the difference (Diff) between the first sensitivity (S1) and the previous sensitivity (Sp) is 40%, which exceeds a specific range (for example, the specific range is 20%). The difference (Diff) may be expressed as the ratio of the difference between the first sensitivity (S1) and the previous sensitivity (Sp) to the first sensitivity (S1), and applying the above values, the difference (Diff) may be 40%. A specific range may be the same concept as a preset threshold. Then, the first sensitivity (S1) may be adjusted to fall within the specific range, where the first sensitivity (S1) may be averaged with the previous sensitivity (Sp). The controller of the terminal may average the first sensitivity (S1) of 25 and the previous sensitivity (Sp) of 35 and adjust the first sensitivity (S1) to 30. This adjusted first sensitivity may be sensitivity A (Sa). Therefore, the sensitivity A (Sa) in the second period (X2) may be 30.

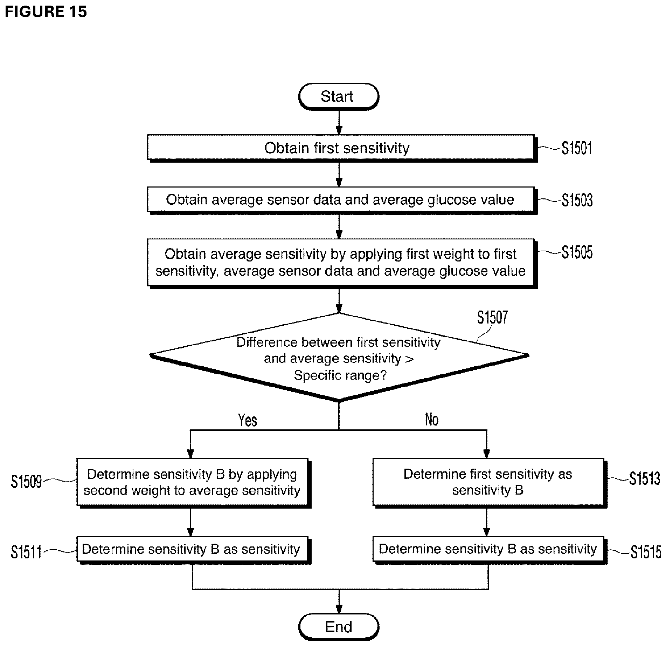

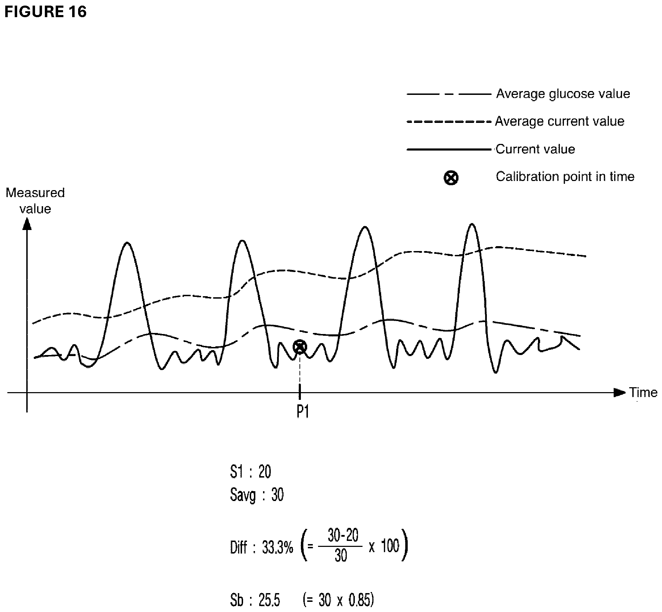

FIG. 15 is a flowchart illustrating a method of adjusting sensitivity based on an average sensor data and an average glucose value according to FIG. 12, and FIG. 16 is a flowchart illustrating a method of adjusting sensitivity based on an average sensor data and an average glucose value according to FIG. 15.

Referring to FIG. 15, a method of adjusting sensitivity based on an average sensor data and an average glucose value, i.e., a method of determining sensitivity B, according to another embodiment, may be shown.

The controller of the terminal may generate a first sensitivity from Equation 2 (step S1501). In addition, the controller of the terminal may obtain an average sensor data and an average glucose value (step S1503). The average sensor data may refer to the value averaged up to the current point in time at which the sensitivity is calibrated, based on the sensor data measured by the sensor. The starting point of the average could be either the point the sensor is inserted or any point in time before the current point in time that has been preset by the user. From that point to the current point in time, sensor data may be averaged. In addition, the average glucose value may refer to the value averaged up to the current point in time at which the sensitivity is calibrated, based on the glucose values obtained in the system. As with the average sensor data, the starting point of the average is determined, and the glucose value may be averaged from that point to the current point in time.

Then, the controller of the terminal may obtain an average sensitivity by applying a first weight to the first sensitivity, average sensor data, and average glucose value (step S1505). The first weight may be assigned to each of the first sensitivity, average sensor data, and average glucose value, and each of the first weights may change over time. For example, the controller of the terminal may apply weight 1a to the first sensitivity, weight 1b to the average sensor data, and weight 1c to the average glucose value. As weights 1b and 1c are adjusted, the ratio reflected in the first sensitivity may increase or decrease.

In addition, the controller of the terminal may calculate the difference between the first sensitivity and the average sensitivity and determine whether the difference exceeds a specific range (step S1507). If the difference exceeds the specific range, the controller of the terminal may determine sensitivity B from the average sensitivity using the average sensitivity instead of the first sensitivity (YES in step S1507 and step S1509). If the first sensitivity is too far from the average sensitivity, the average sensitivity is used. The controller of the terminal may determine sensitivity B by applying a second weight to the average sensitivity. Sensitivity B is determined as the final sensitivity, and the controller of the terminal may obtain the glucose value for the current value through sensitivity B (step S1511).

If the difference does not exceed the specific range, the controller of the terminal may determine the first sensitivity as sensitivity B without using the average sensitivity (NO in step S1507 and step S1513). Sensitivity B is determined as the final sensitivity, and the controller of the terminal may obtain the glucose value for the current value through sensitivity B (step S1515).

Referring to FIG. 16, an example of adjusting sensitivity based on the average sensor data and the average glucose value according to another embodiment may be shown. In this drawing, a current value corresponding to the sensor data, an average current value corresponding to the average sensor data, and an average glucose value may be shown together.

The first calibration point (P1) is the current point in time, and may be the point when the controller of the terminal attempts to obtain the final sensitivity to obtain the glucose value. At the first calibration point (P1), the controller of the terminal may obtain a first sensitivity (S1) of 20. In addition, the controller of the terminal may calculate an average sensitivity (Savg) of 30 by applying a first weight to the average sensor data, average glucose value, and first sensitivity (S1).

In addition, the controller of the terminal may determine sensitivity B (Sb) based on this average sensitivity (Savg). Specifically, the controller of the terminal may obtain the difference (Diff) between the first sensitivity (S1) and the average sensitivity (Savg). The difference (Diff) may be expressed as the ratio of the difference between the first sensitivity (S1) and the average sensitivity (Savg) to the average sensitivity (Savg), and applying the above values, the difference (Diff) may be 33.3%. It may be determined that the difference (Diff) is outside a specific range (for example, the specific range is 20%). The specific range may be the same concept as a preset threshold. Then the average sensitivity (Savg) may be used to determine sensitivity B, and a second weight may be applied to the average sensitivity (Savg). The second weight may be set to 0.85, and sensitivity B may be 25.5. Here, the second weight may change to increase over time. Ultimately, the ratio at which the average sensitivity (Savg) is reflected to sensitivity B (Sb) may increase. This may be because the average sensitivity (Savg) may be more accurate as it reflects past trends over time.

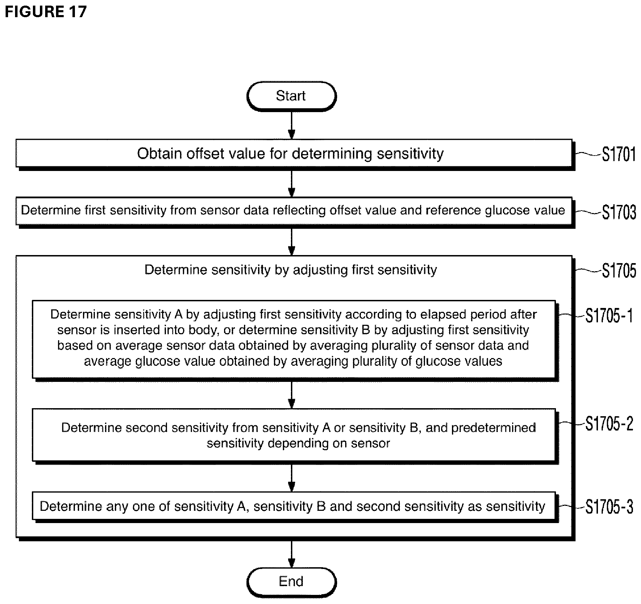

FIG. 17 is a flowchart illustrating a first example of a first method for calibrating sensitivity according to another embodiment.

Referring to FIG. 17, the first method of calibrating sensitivity according to another embodiment may include at least a portion of the sensitivity calibration step described in one embodiment. A first example may be shown in which the first method includes a step of calibrating sensitivity based on a predetermined sensitivity depending on the sensor (i.e., sensor sensitivity).

An offset value for determining sensitivity may be obtained (step S1701). The offset value may be a value that compensates for changes in sensitivity due to a decrease in current value.

In addition, the controller may determine a first sensitivity from the current value reflecting the offset value and the reference glucose value according to Equation 2 (step S1703).

Then, an adjustment to the first sensitivity may be performed. The controller of the terminal may adjust the first sensitivity to finally determine the sensitivity (step S1705). In order to finally determine the sensitivity from the first sensitivity, the controller of the terminal may adjust the first sensitivity in detail as follows.

The controller of the terminal may determine sensitivity A by adjusting the first sensitivity according to the elapsed period of time after the sensor is inserted into the body, or determine sensitivity B by adjusting the first sensitivity based on the average sensor data obtained by averaging the plurality of sensor data and the average glucose value obtained by averaging the plurality of glucose values (step S1705-1).

In addition, the controller of the terminal may determine a second sensitivity from sensitivity A or sensitivity B and a predetermined sensitivity depending on the sensor (step S1705-2). Determining the second sensitivity based on the predetermined sensitivity depending on the sensor may be the same as described above in one embodiment.

The controller of the terminal may determine any one of sensitivity A, sensitivity B, and the second sensitivity as an adjusted first sensitivity, i.e., final sensitivity desired by the controller of the terminal (step S1705-3). Here, in step S1705-1, the controller of the terminal may obtain sensitivity A and sensitivity B, and in step S1705-2, the second sensitivity may be obtained based on any one of sensitivity A and sensitivity B. The controller of the terminal may determine any one of sensitivity A, sensitivity B, and the second sensitivity as the final sensitivity for obtaining the glucose value from the current value. The controller of the terminal may determine the sensitivity by adjusting the first sensitivity through steps S1705-1 to S1705-3.

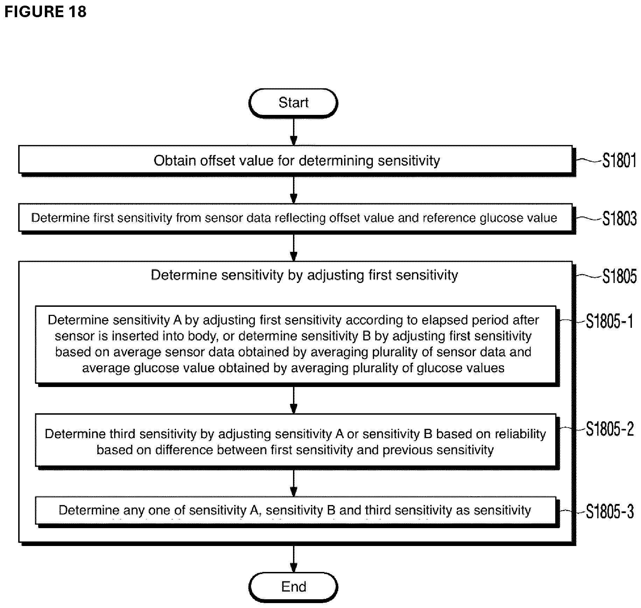

FIG. 18 is a flowchart illustrating a second example of a first method for calibrating sensitivity according to another embodiment.

Referring to FIG. 18, the first method of calibrating sensitivity according to another embodiment may include at least a portion of the sensitivity calibration step described in one embodiment. A second example may be shown in which the first method includes the step of calibrating the sensitivity according to reliability based on the difference between one sensitivity and a previous sensitivity.

An offset value for determining sensitivity may be obtained (step S1801). The offset value may be a value that compensates for changes in sensitivity due to a decrease in current value.

In addition, the controller may determine a first sensitivity from the current value reflecting the offset value and the reference glucose value according to Equation 2 (step S1803).

Then, an adjustment to the first sensitivity may be performed. The controller of the terminal may adjust the first sensitivity to finally determine the sensitivity (step S1805). In order to finally determine the sensitivity from the first sensitivity, the controller of the terminal may adjust the first sensitivity in detail as follows.

The controller of the terminal may determine sensitivity A by adjusting the first sensitivity according to the elapsed period of time after the sensor is inserted into the body, or determine sensitivity B by adjusting the first sensitivity based on the average sensor data obtained by averaging the plurality of sensor data and the average glucose value obtained by averaging the plurality of glucose values (step S1805-1).

In addition, the controller of the terminal may determine a third sensitivity by adjusting sensitivity A or sensitivity B based on reliability based on the difference between the first sensitivity and the previous sensitivity (step S1805-2). Determining the third sensitivity according to reliability based on the difference between the first sensitivity and the previous sensitivity may be the same as described above in one embodiment.

The controller of the terminal may determine any one of sensitivity A, sensitivity B, and the third sensitivity as an adjusted first sensitivity, i.e., final sensitivity desired by the controller of the terminal (step S1705-3). Here, in step S1805-1, the controller of the terminal may obtain sensitivity A and sensitivity B, and in step S1805-2, the third sensitivity may be obtained based on any one of sensitivity A and sensitivity B. The controller of the terminal may determine any one of sensitivity A, sensitivity B, and the third sensitivity as the final sensitivity for obtaining the glucose value from the current value. The controller of the terminal may determine the sensitivity by adjusting the first sensitivity through steps S1805-1 to S1805-3.

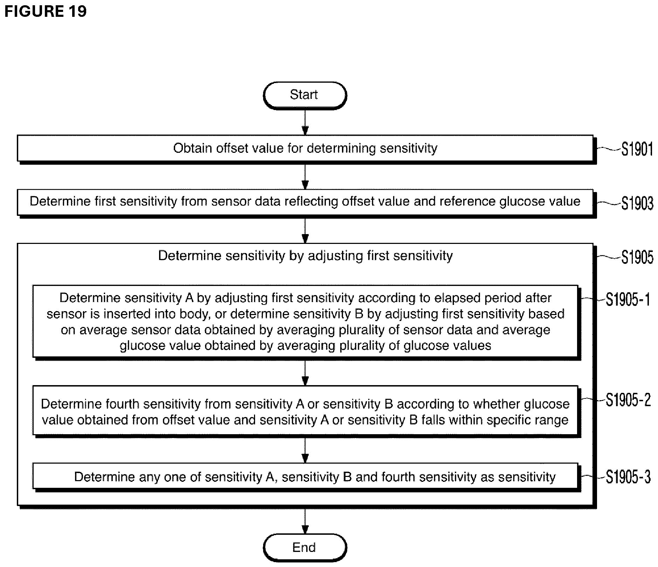

FIG. 19 is a flowchart illustrating a third example of a first method for calibrating sensitivity according to another embodiment.

Referring to FIG. 19, the first method of calibrating sensitivity according to another embodiment may include at least a portion of the sensitivity calibration step described in one embodiment. A third example may be shown in which the first method includes a step of calibrating the sensitivity according to whether the glucose value obtained from the offset value and one sensitivity falls within a specific range.

An offset value for determining sensitivity may be obtained (step S1901). The offset value may be a value that compensates for changes in sensitivity due to a decrease in current value.

In addition, the controller may determine a first sensitivity from the current value reflecting the offset value and the reference glucose value according to Equation 2 (step S1903).

Then, an adjustment to the first sensitivity may be performed. The controller of the terminal may adjust the first sensitivity to finally determine the sensitivity (step S1905). In order to finally determine the sensitivity from the first sensitivity, the controller of the terminal may adjust the first sensitivity in detail as follows.

The controller of the terminal may determine sensitivity A by adjusting the first sensitivity according to the elapsed period of time after the sensor is inserted into the body, or determine sensitivity B by adjusting the first sensitivity based on the average sensor data obtained by averaging the plurality of sensor data and the average glucose value obtained by averaging the plurality of glucose values (step S1905-1).

In addition, the controller of the terminal may determine a fourth sensitivity from sensitivity A or sensitivity B according to whether the glucose value obtained from the offset value and sensitivity A or sensitivity B falls within a specific range (step S1905-2). Determining the fourth sensitivity may be the same as described above in one embodiment.

The controller of the terminal may determine any one of sensitivity A, sensitivity B, and the fourth sensitivity as an adjusted first sensitivity, i.e., final sensitivity desired by the controller of the terminal (step S1905-3). Here, in step S1905-1, the controller of the terminal may obtain sensitivity A and sensitivity B, and in step S1905-2, the fourth sensitivity may be obtained based on any one of sensitivity A and sensitivity B. The controller of the terminal may determine any one of sensitivity A, sensitivity B, and the fourth sensitivity as the final sensitivity for obtaining the glucose value from the current value. The controller of the terminal may determine the sensitivity by adjusting the first sensitivity through steps S1905-1 to S1905-3.

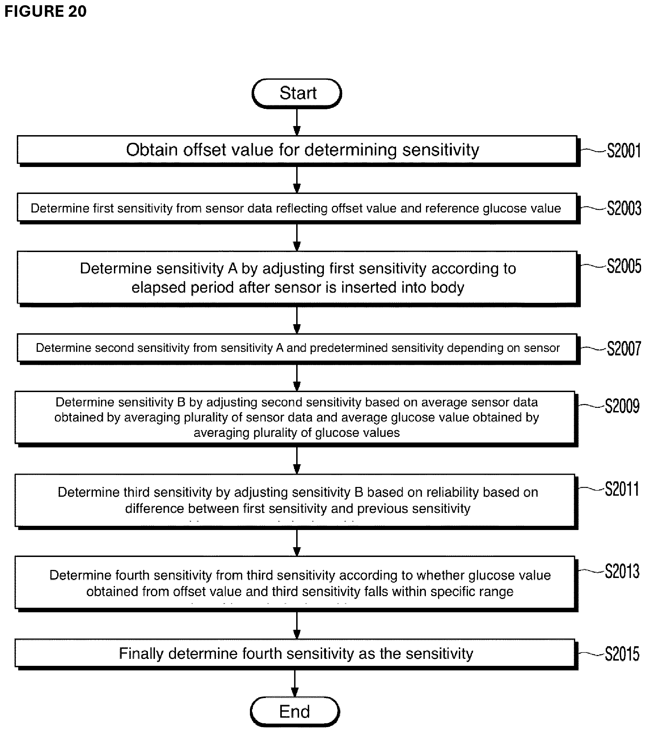

FIG. 20 is a flowchart illustrating a second method for calibrating sensitivity according to another embodiment.

Referring to FIG. 20, a second method of calibrating sensitivity in a terminal that constitutes a system according to another embodiment may be shown. While in the first method each step of determining sensitivity A, sensitivity B and the second to fourth sensitivities may be applied selectively or in parallel, in the second method, each step may be applied serially in one order.

Specifically, an offset value for determining sensitivity may be obtained (step S2001). The offset value may be a value that compensates for changes in sensitivity due to a decrease in current value.

In addition, the controller may determine a first sensitivity from the current value reflecting the offset value and the reference glucose value according to Equation 2 (step S2003).

In addition, the controller of the terminal may obtain the first sensitivity and determine sensitivity A by adjusting the first sensitivity according to the elapsed period of time after the sensor is inserted (step S2005).

In addition, the controller of the terminal may obtain sensitivity A and determine a second sensitivity from sensitivity A and sensor sensitivity (step S2007).

In addition, the controller of the terminal may obtain the second sensitivity, and determine sensitivity B by adjusting the second sensitivity based on the average sensor data obtained by averaging the plurality of sensor data and the average glucose value obtained by averaging the plurality of glucose values (step S2009).

In addition, the controller of the terminal may obtain sensitivity B and determine a third sensitivity by adjusting sensitivity B based on reliability based on the difference between the first sensitivity and a previous sensitivity (step S2011).

In addition, the controller of the terminal may obtain the third sensitivity and determine a fourth sensitivity from the third sensitivity according to whether the glucose value obtained from the offset value and the third sensitivity falls within a specific range (step S2013).

In addition, the controller of the terminal may determine the fourth sensitivity as the final sensitivity (step S2015).

The aspects of the subject matter described herein may be described in the context of computer-executable instructions, such as program modules, that are executed on a computer. Generally, program modules include routines, programs, objects, components, data structures, etc., which perform specific tasks or specific abstract data types.

Alternatively or additionally, the functionality described herein may be performed, at least in part, by one or more hardware logic components. By way of example, and not limitation, exemplary types of hardware logic components that may be used include field-programmable gate array (FPGA), program-specific integrated circuit (ASIC), application-specific standard product (ASSP), system-on-a-chip system (SOC), complex programmable logic device (CPLD), etc.

In addition, the disclosed embodiments may be implemented in the form of a non-transitory recording medium that stores programs and/or instructions executable by a computer. Instructions may be stored in the form of program code, and when executed by a processor, may create program modules to perform operations of the disclosed embodiments. The non-transitory recording medium may be implemented as a non-transitory computer-readable recording medium.

Computer-readable recording media include all types of recording media storing instructions that may be decoded by a computer. For example, there may be read only memory (ROM), random access memory (RAM), magnetic tape, magnetic disk, flash memory, optical data storage, etc.

Although embodiments of the present disclosure have been described above, those skilled in the art will be able to make various modifications and changes to the present disclosure by adding, changing or deleting components, without departing from the spirit of the present disclosure as set forth in the appended claims, which are included within the scope of rights of the present disclosure.

**Claims**

1. A method for calibrating sensitivity for glucose measurement, the method comprising:

   obtaining (S1201) an offset value for determining sensitivity;
   determining (S1203) a first sensitivity from a sensor data and a reference glucose value, based on the offset value; and
   determining (S1205) sensitivity by adjusting the first sensitivity,
   wherein the determining of the sensitivity comprises:

   obtaining the first sensitivity, and determining (S1205-1) sensitivity A by adjusting the first sensitivity according to an elapsed period of time after a sensor is inserted into a body;
   obtaining the first sensitivity, and determining (S1205-1) sensitivity B by adjusting the first sensitivity by reflecting an average sensor data obtained by averaging a plurality of sensor data and an average glucose value obtained by averaging a plurality of glucose values to the first sensitivity; and
determining (S1205-2) any one of the sensitivity A and the sensitivity B as the sensitivity.

2. The method of claim 1, wherein the elapsed period of time includes a first period representing a time up to a certain point after the sensor is inserted into the body and a second period representing a time after the certain point, and
the determining (S1205-1) of the sensitivity A comprises adjusting (S1303) the first sensitivity in different ways in the first period and the second period.

3. The method of claim 2, wherein the determining (S1205-1) of the sensitivity A comprises adjusting (S1309) the first sensitivity so that the first sensitivity becomes smaller if the first sensitivity in the first period is greater than a sensitivity initially determined after the sensor is inserted into the body.

4. The method of claim 2, wherein the determining (S1205-1) of the sensitivity A comprises adjusting the first sensitivity so that the first sensitivity falls within a specific range if a difference between the first sensitivity in the second period and a previous sensitivity falls outside the specific range.

5. The method of claim 4, wherein the determining (S1205-1) of the sensitivity A comprises averaging (S1319) the first sensitivity and the previous sensitivity in order to adjust the first sensitivity.

6. The method of claim 1, wherein the determining (S1205-1) of the sensitivity B comprises:

   obtaining (S1505) a result of applying a first weight to the average sensor data, the average glucose value, and the first sensitivity as an average sensitivity; and
   determining the sensitivity B based on the average sensitivity.

7. The method of claim 6, wherein the determining (S1205-1) of the sensitivity B based on the average sensitivity comprises determining (S1509) a result of applying a second weight to the average sensitivity as the sensitivity B if a difference between the first sensitivity and the average sensitivity falls outside a specific range.

8. The method of claim 7, wherein the second weight increases over time to increase a reflection ratio of the average sensitivity to the sensitivity B.

9. The method of claim 1, wherein the determining (S1205) of the sensitivity comprises obtaining the sensitivity A and the sensitivity B, and determining (S1705-2) a second sensitivity from the sensitivity A and the sensitivity B and a predetermined sensitivity depending on a sensor, and

the determining (S1205-2) of any one of the sensitivity A and the sensitivity B as the sensitivity comprises determining (S1705-3) any one of the sensitivity A, the sensitivity B, and the second sensitivity as the sensitivity.

10. The method of claim 1, wherein the determining (S1205) of the sensitivity comprises obtaining the sensitivity A and the sensitivity B, and determining (S1805-2) a third sensitivity by adjusting the sensitivity A and the sensitivity B according to a reliability of the first sensitivity based on a difference between the first sensitivity and a previous sensitivity, and

the determining (S1205-2) of any one of the sensitivity A and the sensitivity B as the sensitivity comprises determining (S1805-3) any one of the sensitivity A, the sensitivity B, and the third sensitivity as the sensitivity.

11. The method of claim 1, wherein the determining (S1205) of the sensitivity further comprises obtaining the sensitivity A and the sensitivity B, and determining (S1905-2) a fourth sensitivity from the sensitivity A and the sensitivity B according to whether a glucose value obtained from the offset value and the sensitivity A and the sensitivity B falls within a specific range, and

the determining (S1205-2) of any one of the sensitivity A and the sensitivity B as the sensitivity comprises determining (S1905-3) any one of the sensitivity A, the sensitivity B, and the fourth sensitivity as the sensitivity.

12. A method for calibrating sensitivity for glucose measurement, the method comprising:

obtaining (S2001) an offset value for determining sensitivity;
determining (S2003) a first sensitivity from a senor data and a reference glucose value, based on the offset value;
determining (S2005) sensitivity A by adjusting the first sensitivity according to an elapsed period of time after a sensor is inserted into a body;
determining (S2007) a second sensitivity from the sensitivity A and a predetermined sensitivity depending on a sensor;
determining (S2009) sensitivity B by adjusting the second sensitivity by reflecting an average sensor data obtained by averaging a plurality of sensor data over a predetermined period and an average glucose value obtained by averaging a plurality of glucose values over the predetermined period to the second sensitivity;
determining (S2011) a third sensitivity by adjusting the sensitivity B according to a reliability of the first sensitivity based on a difference between the first sensitivity and a previous sensitivity;
determining (S2013) a fourth sensitivity from the third sensitivity according to whether a glucose value obtained from the offset value and the third sensitivity falls within a specific range; and
determining (S2015) the fourth sensitivity as the sensitivity.

**FIGURE 1**

**FIGURE 2**

300

**FIGURE 3**

**FIGURE 4**

<u>100</u>

110

Sensor Module

Sensor
Communication Part

Sensor Controller

Sensor
Storage Part

120

130

140

**FIGURE 5**

<u>200</u>

210

Output Part

Communication
Part

Controller

Storage Part

220

230

240

**FIGURE 6**

```
                         ┌─────────────┐
                         │    Start    │
                         └──────┬──────┘
                                │
    ┌───────────────────────────▼───────────────────────────┐
    │      Obtain offset value for determining sensitivity    │──S601
    └───────────────────────────┬───────────────────────────┘
                                │
    ┌───────────────────────────▼───────────────────────────┐
    │ Determine first sensitivity from sensor data reflecting │──S603
    │       offset value and reference glucose value          │
    └───────────────────────────┬───────────────────────────┘
                                │
    ┌───────────────────────────▼───────────────────────────┐
    │ Determine second sensitivity from first sensitivity and │──S605
    │      predetermined sensitivity depending on sensor      │
    └───────────────────────────┬───────────────────────────┘
                                │
    ┌───────────────────────────▼───────────────────────────┐
    │ Determine third sensitivity by adjusting second         │──S607
    │ sensitivity based on reliability based on               │
    │ difference between first sensitivity and previous        │
    │ sensitivity                                             │
    └───────────────────────────┬───────────────────────────┘
                                │
    ┌───────────────────────────▼───────────────────────────┐
    │ Determine fourth sensitivity from third sensitivity     │──S609
    │ according to whether glucose value                      │
    │ obtained from offset value and third sensitivity falls   │
    │ within specific range                                   │
    └───────────────────────────┬───────────────────────────┘
                                │
    ┌───────────────────────────▼───────────────────────────┐
    │   Finally determine fourth sensitivity as the sensitivity│──S611
    └───────────────────────────┬───────────────────────────┘
                                │
                         ┌──────▼──────┐
                         │     End     │
                         └─────────────┘
```

**FIGURE 7**

**FIGURE 8**

**FIGURE 9**

Current
value

⊗ : Calibration point in time

BGr = 200

P1

Time

C : 7
Sp : 20
Voff_p : 0
BGp : (7-0) x 20 = 140

C : 7
S3 : 30
Voff : 0
BG : (7-0) x 30 = 210

$$S4 : 24.29 \left( = \frac{140 + 200}{2(7-0)} \right)$$

BG=210

BGp=140      BGr=200

**FIGURE 10**

Current value

⊗ : Calibration point in time

BGr = 140

P2

Time

C : 5
Sp : 30
Voff_p : 1
BGp : (5-1) x 30 = 120

C : 5
S3 : 28
Voff : 0.5
BGp : (5-0.5) x 28 = 126

S4 : 28

BG=126

BGp=120     BGr=140

**FIGURE 11**

Current value

⊗ : Calibration point in time

BGr = 200

P3

Time

C : 12
Sp : 25
Voff_p : 0.5
BGp : (12-0.5) x 25 = 287.5

C : 12
S3 : 15
Voff : 0.3
BG : (12-0.3) x 15 = 175.5

$$S4 : 20.33 \left( = \frac{287.5 + 200}{2(12-0.3)} \right)$$

BG=175.5

BGr=200          BGp=287.5

**FIGURE 12**

```
                          ┌──────────┐
                          │  Start   │
                          └──────────┘
                                │
                                ▼
   ┌────────────────────────────────────────────────────────┐
   │      Obtain offset value for determining sensitivity    │──── S1201
   └────────────────────────────────────────────────────────┘
                                │
                                ▼
   ┌────────────────────────────────────────────────────────────────────┐
   │ Determine first sensitivity from sensor data reflecting offset value │──── S1203
   │                  and reference glucose value                         │
   └────────────────────────────────────────────────────────────────────┘
                                │
                                ▼
   ┌────────────────────────────────────────────────────────────────────┐
   │         Determine sensitivity by adjusting first sensitivity         │──── S1205
   │                                                                      │
   │  ┌────────────────────────────────────────────────────────────────┐ │
   │  │ Determine sensitivity A by adjusting first sensitivity according │ │── S1205-1
   │  │ to elapsed period after sensor is inserted into body, or         │ │
   │  │ determine sensitivity B by adjusting first sensitivity based on   │ │
   │  │ average sensor data obtained by averaging plurality of sensor    │ │
   │  │ data and average glucose value obtained by averaging plurality   │ │
   │  │ of glucose values                                                │ │
   │  └────────────────────────────────────────────────────────────────┘ │
   │                                │                                      │
   │                                ▼                                      │
   │  ┌────────────────────────────────────────────────────────────────┐ │
   │  │  Determine any one of sensitivity A or sensitivity B as          │ │── S1205-2
   │  │                      sensitivity                                 │ │
   │  └────────────────────────────────────────────────────────────────┘ │
   └────────────────────────────────────────────────────────────────────┘
                                │
                                ▼
                          ┌──────────┐
                          │   End    │
                          └──────────┘
```

**FIGURE 13**

**FIGURE 14**

⊗ Calibration point in time

Measured value

Current value

X1 First period    X2 Second period

P1    P2 P3    P4    P5    Time

Si : 50

S1 : 55    Sp : 35    S1 : 25
Si : 50
Sa : 50(55 -> 50)    Diff : 40%    $\left( = \dfrac{35-25}{25} \times 100 \right)$

Sa : 30    $\left( = \dfrac{25+25}{2} \right)$

**FIGURE 15**

```
                              ┌──────────┐
                              │  Start   │
                              └────┬─────┘
                                   ▼
        ┌──────────────────────────────────────────────┐
        │         Obtain first sensitivity             │──── S1501
        └──────────────────────┬───────────────────────┘
                               ▼
        ┌──────────────────────────────────────────────┐
        │  Obtain average sensor data and average glucose value │──── S1503
        └──────────────────────┬───────────────────────┘
                               ▼
        ┌──────────────────────────────────────────────┐  S1505
        │ Obtain average sensitivity by applying first weight to first │
        │ sensitivity, average sensor data and average glucose value   │
        └──────────────────────┬───────────────────────┘
                               ▼
                          ╱──────────╲              S1507
                        ╱  Difference between first  ╲
                       ◇   sensitivity and average   ◇
                        ╲  sensitivity >             ╱
                         ╲  Specific range?        ╱
                           ╲──────────╱
                     Yes    │              │    No
              ┌─────────────┘              └─────────────┐
              ▼                                          ▼
   ┌────────────────────────────┐         ┌────────────────────────────┐
   │ Determine sensitivity B by │         │ Determine first sensitivity │
S1509 │ applying second weight to │       │   as sensitivity B          │── S1513
   │ average sensitivity        │         └─────────────┬──────────────┘
   └─────────────┬──────────────┘                       ▼
                 ▼                          ┌────────────────────────────┐
   ┌────────────────────────────┐           │ Determine sensitivity B as │── S1515
S1511 │ Determine sensitivity B as │         │       sensitivity          │
   │        sensitivity         │           └─────────────┬──────────────┘
   └─────────────┬──────────────┘                         │
                 └──────────────┬─────────────────────────┘
                                ▼
                          ┌──────────┐
                          │   End    │
                          └──────────┘
```

**FIGURE 16**

——— — ——— Average glucose value

------------- Average current value

——————— ·Current value

⊗    Calibration point in time

Measured
value

Time

P1

S1 : 20

Savg : 30

$$\text{Diff} : 33.3\% \left( = \frac{30\text{-}20}{30} \times 100 \right)$$

Sb : 25.5    (= 30 x 0.85)

**FIGURE 17**

```
                          ┌─────────────┐
                          │    Start    │
                          └──────┬──────┘
                                 │
   ┌─────────────────────────────▼──────────────────────────────┐
   │         Obtain offset value for determining sensitivity     │──── S1701
   └─────────────────────────────┬──────────────────────────────┘
                                 │
   ┌─────────────────────────────▼──────────────────────────────┐
   │ Determine first sensitivity from sensor data reflecting      │──── S1703
   │ offset value and reference glucose value                     │
   └─────────────────────────────┬──────────────────────────────┘
                                 │
   ┌─────────────────────────────▼──────────────────────────────┐
   │          Determine sensitivity by adjusting first sensitivity │──── S1705
   │                                                               │
   │  ┌──────────────────────────────────────────────────────┐   │
   │  │ Determine sensitivity A by adjusting first sensitivity│   │──── S1705-1
   │  │ according to elapsed period after sensor is inserted  │   │
   │  │ into body, or determine sensitivity B by adjusting    │   │
   │  │ first sensitivity based on average sensor data obtained│   │
   │  │ by averaging plurality of sensor data and average     │   │
   │  │ glucose value obtained by averaging plurality of      │   │
   │  │ glucose values                                        │   │
   │  └──────────────────────────┬───────────────────────────┘   │
   │                             │                                 │
   │  ┌──────────────────────────▼───────────────────────────┐   │
   │  │ Determine second sensitivity from sensitivity A or     │   │──── S1705-2
   │  │ sensitivity B, and predetermined sensitivity           │   │
   │  │ depending on sensor                                    │   │
   │  └──────────────────────────┬───────────────────────────┘   │
   │                             │                                 │
   │  ┌──────────────────────────▼───────────────────────────┐   │
   │  │ Determine any one of sensitivity A, sensitivity B and  │   │──── S1705-3
   │  │ second sensitivity as sensitivity                      │   │
   │  └──────────────────────────────────────────────────────┘   │
   └─────────────────────────────┬──────────────────────────────┘
                                 │
                          ┌──────▼──────┐
                          │     End     │
                          └─────────────┘
```

**FIGURE 18**

```
                    ┌─────────────┐
                    │    Start    │
                    └──────┬──────┘
                           │
   ┌───────────────────────▼────────────────────────────────┐
   │        Obtain offset value for determining sensitivity  │──S1801
   └───────────────────────┬────────────────────────────────┘
                           │
   ┌───────────────────────▼────────────────────────────────────────────┐
   │ Determine first sensitivity from sensor data reflecting offset      │──S1803
   │ value and reference glucose value                                   │
   └───────────────────────┬────────────────────────────────────────────┘
                           │
   ┌───────────────────────▼────────────────────────────────────────────┐
   │        Determine sensitivity by adjusting first sensitivity         │──S1805
   │  ┌──────────────────────────────────────────────────────────────┐   │
   │  │ Determine sensitivity A by adjusting first sensitivity        │   │──S1805-1
   │  │ according to elapsed period after sensor is inserted into     │   │
   │  │ body, or determine sensitivity B by adjusting first           │   │
   │  │ sensitivity based on average sensor data obtained by          │   │
   │  │ averaging plurality of sensor data and average glucose        │   │
   │  │ value obtained by averaging plurality of glucose values       │   │
   │  └──────────────────────────┬───────────────────────────────────┘   │
   │  ┌──────────────────────────▼───────────────────────────────────┐   │
   │  │ Determine third sensitivity by adjusting sensitivity A or     │   │──S1805-2
   │  │ sensitivity B based on reliability based on difference        │   │
   │  │ between first sensitivity and previous sensitivity            │   │
   │  └──────────────────────────┬───────────────────────────────────┘   │
   │  ┌──────────────────────────▼───────────────────────────────────┐   │
   │  │ Determine any one of sensitivity A, sensitivity B and third   │   │──S1805-3
   │  │ sensitivity as sensitivity                                    │   │
   │  └──────────────────────────────────────────────────────────────┘   │
   └───────────────────────┬─────────────────────────────────────────────┘
                           │
                    ┌──────▼──────┐
                    │     End     │
                    └─────────────┘
```

**FIGURE 19**

Start

Obtain offset value for determining sensitivity — S1901

Determine first sensitivity from sensor data reflecting offset value and reference glucose value — S1903

Determine sensitivity by adjusting first sensitivity — S1905

Determine sensitivity A by adjusting first sensitivity according to elapsed period after sensor is inserted into body, or determine sensitivity B by adjusting first sensitivity based on average sensor data obtained by averaging plurality of sensor data and average glucose value obtained by averaging plurality of glucose values — S1905-1

Determine fourth sensitivity from sensitivity A or sensitivity B according to whether glucose value obtained from offset value and sensitivity A or sensitivity B falls within specific range — S1905-2

Determine any one of sensitivity A, sensitivity B and fourth sensitivity as sensitivity — S1905-3

End

**FIGURE 20**

```
                    ┌──────────┐
                    │  Start   │
                    └────┬─────┘
                         ▼
┌──────────────────────────────────────────────────────────┐
│      Obtain offset value for determining sensitivity        │──S2001
└────────────────────────┬─────────────────────────────────┘
                         ▼
┌──────────────────────────────────────────────────────────┐
│ Determine first sensitivity from sensor data reflecting offset value and reference glucose value │──S2003
└────────────────────────┬─────────────────────────────────┘
                         ▼
┌──────────────────────────────────────────────────────────┐
│      Determine sensitivity A by adjusting first sensitivity according to │──S2005
│           elapsed period after sensor is inserted into body │
└────────────────────────┬─────────────────────────────────┘
                         ▼
┌──────────────────────────────────────────────────────────┐
│ Determine second sensitivity from sensitivity A and predetermined sensitivity depending on sensor │──S2007
└────────────────────────┬─────────────────────────────────┘
                         ▼
┌──────────────────────────────────────────────────────────┐
│ Determine sensitivity B by adjusting second sensitivity based on average sensor data │──S2009
│ obtained by averaging plurality of sensor data and average glucose value obtained by │
│             averaging plurality of glucose values │
└────────────────────────┬─────────────────────────────────┘
                         ▼
┌──────────────────────────────────────────────────────────┐
│ Determine third sensitivity by adjusting sensitivity B based on reliability based on │──S2011
│        difference between first sensitivity and previous sensitivity │
└────────────────────────┬─────────────────────────────────┘
                         ▼
┌──────────────────────────────────────────────────────────┐
│ Determine fourth sensitivity from third sensitivity according to whether glucose value │──S2013
│   obtained from offset value and third sensitivity falls within specific range │
└────────────────────────┬─────────────────────────────────┘
                         ▼
┌──────────────────────────────────────────────────────────┐
│      Finally determine fourth sensitivity as the sensitivity │──S2015
└────────────────────────┬─────────────────────────────────┘
                         ▼
                    ┌──────────┐
                    │   End    │
                    └──────────┘
```

Europäisches
Patentamt

European
Patent Office

Office européen
des brevets

# EUROPEAN SEARCH REPORT

Application Number

EP 24 19 6008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/268477 A1 (MUELLER JR JOHN C [US] ET AL) 21 October 2010 (2010-10-21) * para. 89-114, 140, figures 13-15 * | 1-12 | INV. A61B5/145 A61B5/1495 A61B5/1486 |
| A | US 2020/121231 A1 (HAYTER GARY A [US]) 23 April 2020 (2020-04-23) * the whole document * | 1-12 | A61B5/00 |

TECHNICAL FIELDS
SEARCHED      (IPC)

A61B

The present search report has been drawn up for all claims

| Place of search | Date of completion of the search | Examiner |
|---|---|---|
| The Hague | 7 January 2025 | Horrix, Doerte |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 24 19 6008

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2025

| Patent document cited in search report | Publication date | Patent family member(s) | | Publication date |
|---|---|---|---|---|
| US 2010268477 A1 | 21-10-2010 | CA | 2702428 A1 | 07-05-2009 |
| | | CA | 2782491 A1 | 07-05-2009 |
| | | DK | 2207473 T3 | 15-06-2015 |
| | | EP | 2207473 A2 | 21-07-2010 |
| | | EP | 2896357 A1 | 22-07-2015 |
| | | US | 2009112478 A1 | 30-04-2009 |
| | | US | 2010268477 A1 | 21-10-2010 |
| | | WO | 2009058792 A2 | 07-05-2009 |
| US 2020121231 A1 | 23-04-2020 | US | 2009036760 A1 | 05-02-2009 |
| | | US | 2017181679 A1 | 29-06-2017 |
| | | US | 2020121231 A1 | 23-04-2020 |
| | | US | 2022273203 A1 | 01-09-2022 |
| | | US | 2023293057 A1 | 21-09-2023 |

EPO FORM P0459

For more details about this annex : see Official Journal of the European Patent Office, No. 12/82

**EP 4 512 327 A1**

**Patent documents cited in the description**

- KR 1020230110959